# EUROPÄISCHE PATENTSCHRIFT

(11) **EP 2 806 830 B1**
(45) Veröffentlichungstag und Bekanntmachung des Hinweises auf die Patenterteilung: **15.06.2016**
(21) Anmeldenummer: 13707536.2
(22) Anmeldetag: 17.01.2013
(51) Int. Cl.: A61F 2/36, A61F 2/46, A61B 17/72, A61F 2/30

(54) **ADAPTER-SYSTEM FÜR EINE ENDOPROTHESE**
ADAPTER SYSTEM FOR AN ENDOPROSTHESIS
SYSTÈME D'ADAPTATEUR POUR ENDOPROTHÈSE

(30) Priorität: 26.01.2012 DE 102012001395
(43) Veröffentlichungstag der Anmeldung: 03.12.2014
(73) Patentinhaber: Merete Medical GmbH, 12247 Berlin (DE)
(72) Erfinder: ANAPLIOTIS, Emmanuel, 14295 Berlin (DE); LOB, Guenter, 81377 München (DE)
(74) Vertreter: Hannig, Wolf-Dieter
(86) Internationale Anmeldenummer: PCT/DE2013/000032
(87) Internationale Veröffentlichungsnummer: WO 2013/110259

(56) Entgegenhaltungen:
- EP-A1- 0 145 641
- EP-A1- 0 567 349
- EP-A1- 1 913 902
- WO-A1-2010/034284
- DE-A1- 3 205 577
- DE-A1-102008 062 226
- US-A- 4 938 768

## Beschreibung

Die Erfindung betrifft ein periprothetisches Adapter-System für einen bereits implantierten Schaft einer Endoprothese.

### Stand der Technik

Es ist hinlänglich bekannt, dass postoperative periprothetische Frakturen sowohl bei gelockerter als auch bei fest sitzenden Endoprothesen auftreten können.
Durch eine progrediente Knochenresorption kommt es oftmals zu einer Aufweitung der Markhöhle, wodurch günstige Biegemomente zwischen dem Schaft und der Prothesenspitze entstehen, die eine Lockerung der Prothese zur Folge haben und Ermüdungsbrüche sowie Frakturen begünstigen können.
Ursachen für Frakturen bei fest sitzender Prothese sind beispielsweise schwerere Traumatisierungen infolge von Stürzen im Haushalt, Verkehrsunfälle, Stürze aus größerer Höhe, unkontrollierte Stürze aufgrund von Nebenerkrankungen oder Revisionseingriffe.

Aus der DE 10 2008 062 226 A1 ist eine Vorrichtung zur Versorgung von Frakturen des Femur mit einem retrograd in den Markraum des Femur einbringbaren distalen Femurnagel bekannt, dessen proximales Ende an das distale Ende eines im Femur bereits vorhandenen proximalen Femurnagels oder eines Hüftschaftes einer Hüftgelenkprothese starr ankoppelbar ist. Der distale Femurnagel weist an seinem proximalen Ende eine Aufnahmeöffnung zur Aufnahme des distalen Endes des proximalen Femurnagels oder des Hüftschaftes auf. Zur starren Ankopplung besitzt der distale Femurnagel im Bereich der Aufnahmeöffnung wenigstens eine erste Durchgangsbohrung zur Verriegelung des distalen Femurnagels am Femur und/oder an dem distalen Ende des proximalen Femurnagels oder des Hüftschaftes mittels einer Verriegelungsschraube auf.
Dieser bekannte Stand der Technik hat den Nachteil, dass eine vollständige Revision des ansonsten fest sitzenden Hüftschaftes der Prothese mit all seinen Beeinträchtigungen für den Patienten durchgeführt werden muss, damit ein mit einer entsprechend eingebrachten Durchgangsbohrung versehener Hüftschaft zur starren Ankopplung des distalen Femurnagels bereitsteht.
Des Weiteren können bei einer späteren Revision erhebliche Probleme bei der Lösung der starren Ankopplung auftreten, weil sich die Verriegelungsschrauben nur nach Extension von Weich-, Muskel- und Sehnenteilen mit entsprechender Traumatisierung des Patienten entfernen lassen.

Des Weiteren ist aus der US 4 938 768 A ein Verbindungsmittel für in Knochen eingesetzte Nägel in Form von aneinander fixierbaren Halbschalen bekannt. Dieses bekannte Verbindungsmittel wird jedoch nicht zum kraftschlüssigen Verbinden eines Schaftes einer Endoprothese mit einem Nagel eingesetzt.

Die EP 0 145 641 A1 beschreibt einen Bausatz für eine Resektionsprothese mit einem Kopfteil und einem Endteil, von denen das eine Teil einen konischen Zapfen und das andere Teil eine konische Bohrung aufweist, wobei zwischen diesen beiden Teilen mindestens ein an Zapfen und/oder Bohrung angepasstes Zwischenteil vorgesehen ist, und die beim Zusammensetzen aneinanderstoßenden Flächen unterschiedlicher Teile durch aneinander angepasste Ausformungen gegen Verdrehen sicherbar sind.

Die EP 0 567 349 A1 offenbart eine modulare Hüftprothese mit einem Hüftprothesenkörper, der umfasst:
a) einen oberen Endabschnitt mit einen an einen runden Kopfabschnitt angepassten Hals, welcher in die Hüftpfanne des Patienten eingesetzt ist, einem Mittelabschnitt mit vorderen und hinteren Seiten und einem unteren Schaftabschnitt zum angepassten Einsetzen in den intramedullären Kanal des Femur des Patienten,
b) entfernbare bezüglich am Hals und Schaft proximale und distale befestigbare Verlängerungselemente zum selektiven Anpassen der Prothese an die benachbarte Gelenkpfanne und das Oberschenkelknochengewebe durch selektives Erhöhen des Prothesenhals,
c) Haltemittel für jedes Teil zum Befestigen des entsprechenden Teils am Prothesenkörper, wobei die Haltemittel entsprechende kegelstumpfförmige Flächen an jedem Verlängerungselement und am Prothesenkörper aufweisen, und
d) wobei der Prothesenkörper eine erste kegelstumpfförmige Fläche auf dem Prothesenhals und eine zweite kegelstumpfförmige Fläche am oberen Endabschnitt des Schaftes hat.

Weiterhin ist aus der WO 2010/034284A1 eine modulare Gelenkprothese mit einem gekrümmt ausgebildeten Kopfteil, auf dessen konischen Zapfen eine aufsteckbare Gelenkkugel befestigt ist, und mit einem in den Knochenmarkkanal des os longum einsetzbaren Nagel mit einem Nagelhals, wobei zwischen Kopfteil und Nagelhals ein Verbindungsteil vorgesehen ist, das das Kopfteil verdrehsicher hält. Das Verbindungsmittel umfasst mindestens ein in Achsrichtung des Nagels gelegenes manschettenartiges Modul mit mindestens zwei in Achsrichtung des Moduls geteilte, lösbar miteinander verbundenen Halbschalen.

### Aufgabenstellung

Bei diesem Stand der Technik liegt der Erfindung die Aufgabe zugrunde, ein periprothetisches Adapter-System für einen bereits implantierten Schaft einer Endoprothese anzugeben, das den Sitz des Schaftes der Prothese im Knochen stabilisiert und zugleich die postoperative Versorgung von periprothetischen Frakturen unter Reduzierung der Belastungen für den Patienten verbessert wird.

Diese Aufgabe wird durch das erfindungsgemäße Adapter-System der eingangs genannten Art mit den Merkmalen des Anspruches 1 gelöst.

Vorteilhafte Ausgestaltungen des Adapter-Systems sind den Unteransprüchen entnehmbar.

Grundgedanke der erfindungsgemäßen Lösung ist es, den im Knochen befindlichen Schaft der Endoprothese durch das Adapter-System mit einem im Knochen eingebrachten distal verriegelbaren Nagel zu verbinden und dadurch die Verankerungslänge des Schaftes im Knochen wirksam zu erhöhen. Dies gelingt durch das Adapter-System, welches umfasst:
a) einen Satz einer Vielzahl von zylindrischen, hohl ausgebildeten Probehülsen mit jeweils unterschiedlichen, wenigstens hälftig in Richtung Zylinderachse angeschnittenen Innenraumformen zum Ermitteln der mit der Außenform des distalen Endes des Schaftes weitgehend übereinstimmenden Geometrie,
b) einen Satz von distal geschlossenen, aber proximal offenen mit dem Satz von Probehülsen mindestens in der Innenraumform übereinstimmenden Implantatshülsen zur Auswahl der geeigneten Implantatshülse für die Aufnahme und eine kraftschlüssige Einbettung des distalen Endes des Schaftes in die Implantatshülse mittels Knochenzement,
c) einen in den Knochen eingesetzten Nagel mit einem zylindrischen Nagelhals und
c) ein Verbindungsmittel für das in die Implantatshülse einzementierte Ende des Schaftes und den Nagel in Form eines an sich bekannten Moduls aus mindestens zwei in Achsrichtung des Moduls geteilte, lösbar miteinander verbundene Halbschalen, die miteinander eine sich axial erstreckende Durchführung zum Einführen des distalen Endes der Implantatshülse und des proximalen Nagelhalses ausbilden, wobei die Halbschalen durch senkrecht zur Achsrichtung positionierte Spannmittel den Nagelhals und die Implantatshülse in der Durchführung spannbackenartig fixieren.

Dies ist mit dem außerordentlichen Vorteil verbunden, dass insbesondere Frakturen im Bereich der Schaftspitze ohne Revision des Schaftes versorgt werden können.

Aus der Vielzahl von Probehülsen wird diejenige Hülse durch Aufsetzen der hohlen Probehülse auf das Schaftende ermittelt, die einen nahen Formschluß der Außenform des distalen Endes des Schaftes mit der Innenform des Hohlraumes der Probehülse gewährleistet, und dadurch die geeignete Implantatshülse bestimmt, in die das Schaftende eingesetzt und einzementiert wird.

Von besonderem Vorteil ist weiterhin, wenn die Außendurchmesser der ausgewählten Implantatshülse und des zylindrischen Nagelhalses an den jeweiligen Innendurchmesser der aus den beiden Halbschalen des Verbindungsmittels gebildeten Durchführung angepasst ist. Es ist aber auch möglich, den Außendurchmesser des Nagelhalses auf den Innendurchmesser der Durchführung durch eine auf den zylindrischen Nagelhals aufgeschobene und fixierte Reduzierhülse einzustellen, ohne die Erfindung zu verlassen.

In einer weiteren bevorzugten Ausgestaltung des erfindungsgemäßen Adapter-Systems sind die Innenräume der Probe- und Implantatshülsen eines Satzes mit Innenraumformen versehen, die in Form und Geometrie an das Schaftende handelsüblicher Endoprothesen angepasst sind.
Die Innenraumform kann vorzugsweise aus zylindrischen, kegel-, kegelstumpf-, parabel-, kreissegment-, halbkugelförmigen Flächen oder aus entsprechend zusammengesetzten Flächen gebildet sein, wodurch es möglich wird, die verschiedenartig ausgebildeten Schaftenden unterschiedlicher Prothesen an das erfindungsgemäße Adapter-System kraftschlüssig anzudocken.

In einer weiteren bevorzugten Ausführungsform der Erfindung haben die Implantatshülsen unterschiedlich axiale Längen, Außendurchmesser und Innenraumformen, denen jeweils deckungsgleiche Probehülsen zum Ermitteln eines formschlußnahen Sitzes des Schaftendes in der Implantatshülse zugeordnet sind.
Dies stellt sicher, dass der Chirurg die geeignete Implantatshülse schnell und problemlos während der Operation feststellen kann.

Bei einer weiteren vorteilhaften Weiterbildung der Erfindung sind neben den Implantatshülsen auch die Module des Verbindungsmittels längenvariabel, so dass das erfindungsgemäße Adapter-System gut an die verschiedenartigen Bedingungen der einzelnen Patienten anpassbar ist und eine individuell deutlich vergrößerte Längenvariabilität für die Versorgung periprothetischer Frakturen besitzt.
Diese Längenvariabilität kann weiter dadurch verbessert werden, wenn zum Verbinden von Modulen gleicher oder unterschiedlicher Länge ein Modulverbinder vorgesehen wird, dessen Außendurchmesser auf den Innendurchmesser der Durchführung abgestimmt ist.

In einer weiteren Ausgestaltung der Erfindung besitzen alle Flächen der Implantatshülsen, vorzugsweise die Flächen der Innenraumform, eine raue Oberfläche zum Verbessern der Haftkraft des Knochenzements bei Einzementieren des Schaftendes der Prothese in die Implantatshülse.

Alle Teile des erfindungsgemäßen Adapter-Systems bestehen aus einem körperverträglichen und beständigen, vorzugsweise metallischen Werkstoff, beispielsweise Titan, Tantal, Niob oder deren Legierungen bzw. moderne Implantatskunststoffe wie Peek.

Weitere Vorteile und Einzelheiten ergeben sich aus der nachfolgenden Beschreibung unter Bezugnahme auf die beigefügten Zeichnungen.

### Ausführungsbeispiel

Die Erfindung soll nachstehend an einem Ausführungsbeispiel näher erläutert werden.

### Es zeigen

Fig. 1 eine Explosionsdarstellung des erfindungsgemäßen Adapter-Systems,
Fig. 2a bis 2c eine Vorderansicht der Prüfhülsen mit Beispielen unterschiedlicher Innenraumformen,
Fig. 3a bis 3c eine Draufsicht gemäß den Figuren 2a bis 2c,
Fig. 4 eine Seitenansicht einer Probehülse,
Fig. 5a und 5b eine Seitenansicht bzw. Draufsicht einer Implantatshülse mit einem distal angeordneten Steckzapfen, der einen gegenüber der Implantatshülse geringeren Durchmesser aufweist,
Fig. 6a bis 6c einen Schnitt gemäß Linie A-A in Fig. 5b mit verschiedenen Innenraumformen,
Fig. 7a bis 7c eine Schnittdarstellung einer zylindrischen Implantatshülse mit verschiedenen Innenraumformen,
Fig. 8 eine perspektivische Darstellung des erfindungsgemäßen Adapter-Systems im zusammengesetzten Zustand,
Fig. 9 eine perspektivische Darstellung des in Form eines Modules aus zwei Halbschalen zusammengesetzten Verbindungsmittels mit Innenanschlägen für das Schaftende der Prothese und den zylindrischen Nagelhals des Nagels,
Fig. 10a und 11a eine Seitenansicht, Fig. 10b und 11b eine Schnittdarstellung des erfindungsgemäßen Adapter-Systems mit einem gemeinsamen Anschlag für die Implantatshülse und den zylindrischen Nagelhals.

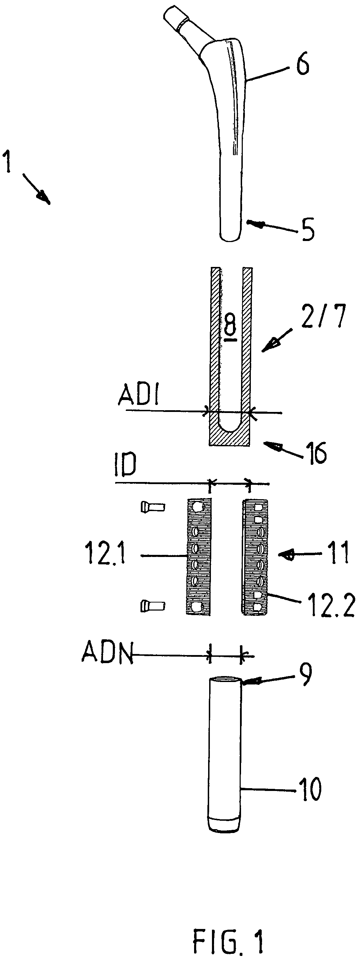

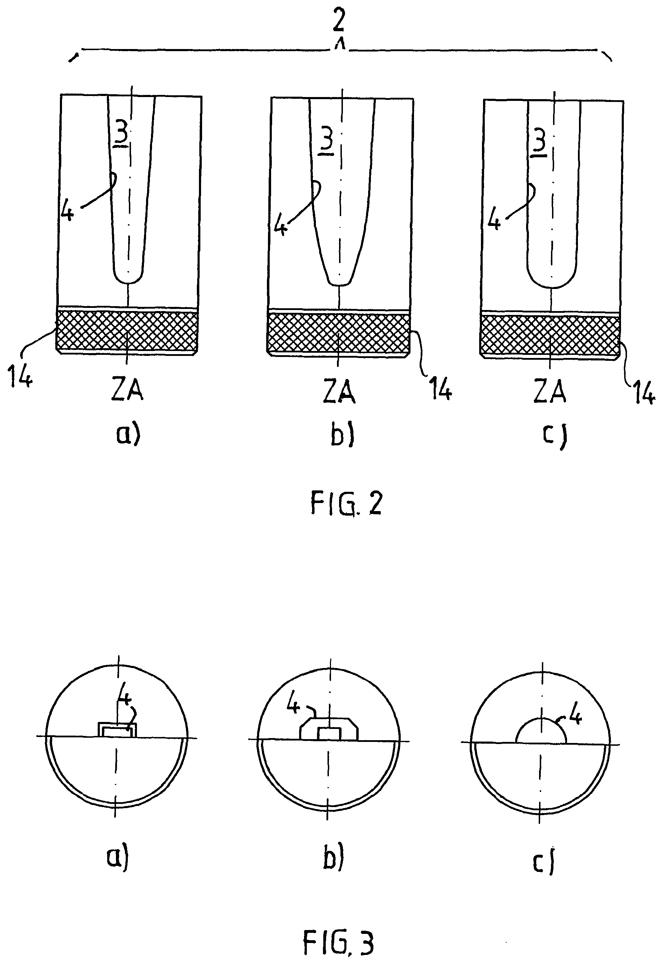

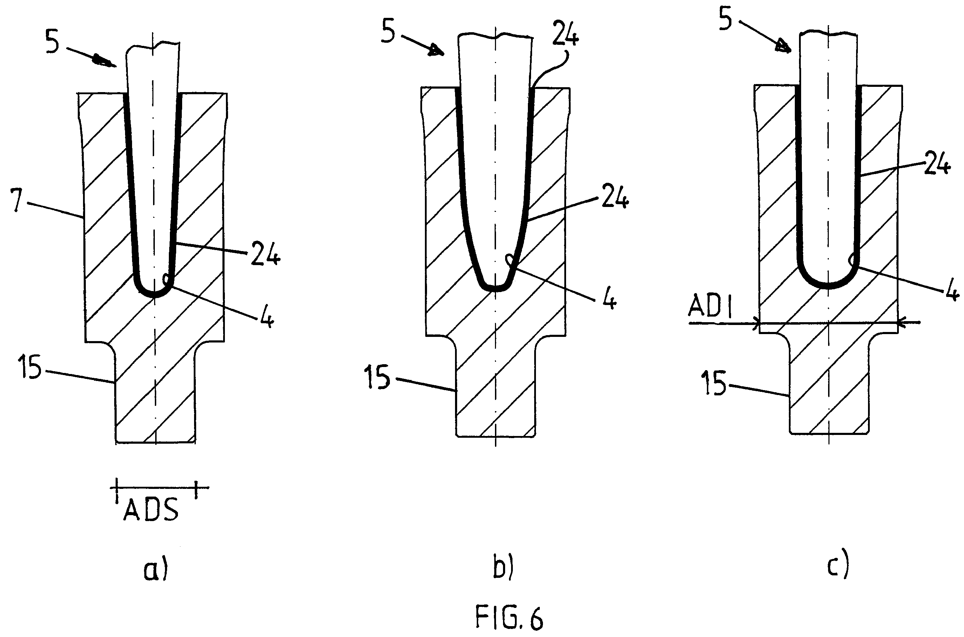

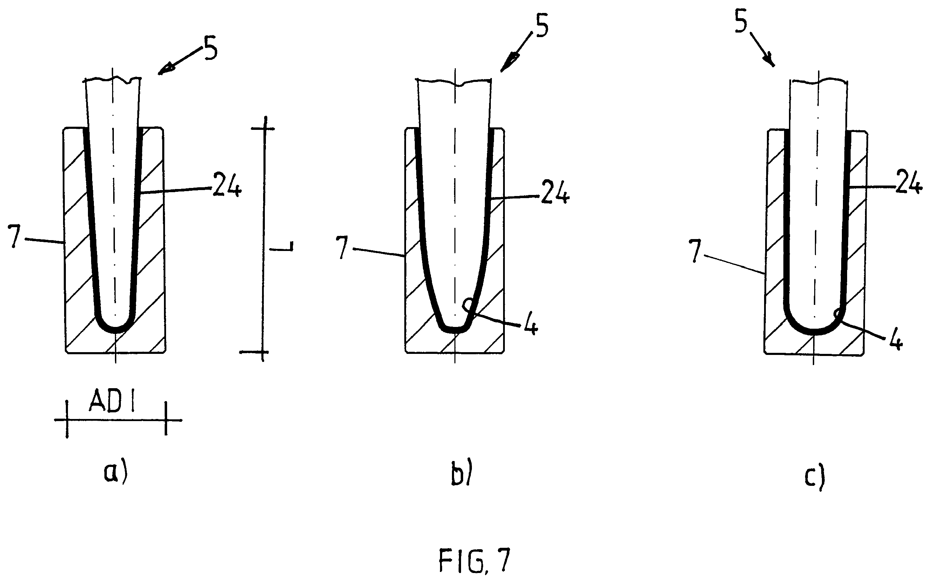

Die Fig. 1 zeigt den grundsätzlichen Aufbau des erfindungsgemäßen Adapter-Systems 1 zum Versorgen einer periprothetischen Fraktur.
Das erfindungsgemäße Adapter-System 1 umfasst - wie beispielsweise in Fig. 2a bis 2c dargestellt- einen Satz einer Vielzahl von halbzylindrisch aufgeschnittenen, distal geschlossenen Probehülsen 2, welche einen proximalseitig zugänglichen Innenraum 3 mit von Probehülse zu Probehülse verschiedenen Innenraumformen 4 besitzen. Zum Ermitteln derjenigen Probehülse 2, die einen formschlußnahen Sitz des Endes 5 des Schaftes 6 im Innenraum 3 der Probehülse gewährleistet, werden die Probehülsen auf das Ende 5 des Schaftes 6 der Endoprothese geschoben und die Probehülse mit der Außenform des distalen Endes des Schaftes weitgehend übereinstimmende Geometrie festgestellt.
Jedem Satz von Probehülsen 2 ist ein Satz einer Vielzahl von Implantatshülsen 7 mit deckungsgleich an die Innenraumformen 4 der Probehülsen 2 angepassten Innenräumen 8 zugeordnet, so dass der Chirurg schnell und problemlos die entsprechend geeignete Implantatshülse 7 auswählen kann (siehe Fig. 6a bis 6c, 7a bis 7c).
Die so ermittelte Implantatshülse 7 wird auf das distale Ende 5 des Hüftschaftes 6 aufgesetzt und das distale Ende 5 mittels Knochenzement im Innenraum 8 der Implantatshülse 7 einzementiert.
Das erfindungsgemäße Adapter-System 1 umfasst weiterhin einen mit einem zylindrischen Nagelhals 9 versehenen Nagel 10, der in den Knochenmarkkanal des nicht dargestellten Knochens eingebracht und verankert wird, und ein Verbindungsmittel 11 in Form eines Moduls, das aus zwei gegen die Implantatshülse 7 und den Nagelhals 9 mittels Spannmittel spannende Halbschalen 12.1 und 12.2 gebildet ist. Diese Halbschalen 12.1 und 12.2 definieren gemeinsam eine in Achsrichtung AR der Halbschalen verlaufende Durchführung 13, in deren proximalen Seite die Implantatshülse 7 und in deren distalen Seite der Nagelhals 9 des Nagels 10 einliegt.
Der Außendurchmesser ADI des in der Durchführung 13 einliegenden Teils der Implantatshülse 7 ist auf den Innendurchmesser ID der Durchführung 13 und ebenso ist der Außendurchmesser ADN des Nagelhalses 9 des Nagels 10 auf den Innendurchmesser ID der Durchführung 13 abgestimmt.
Die Verankerung des Nagels 10 kann durch Verriegelungsschrauben oder Einzementieren mit Knochenzement im Knochen erfolgen. Die Nägel 10 sind längen- und durchmesservariabel ausgebildet und können so an die individuellen Bedingungen beim Patienten angepasst werden.

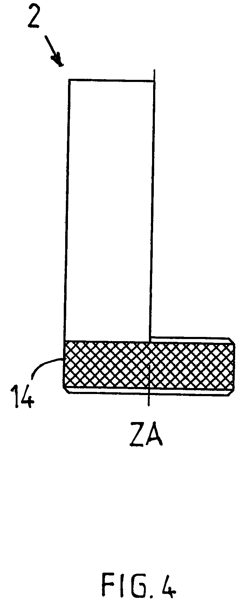

Die Fig. 2a bis 2c, 3a bis 3c und Fig. 4 zeigen Beispiele von Probehülsen 2 mit verschiedenen im Innenraum 3 ausgeformten Innenraumformen 4, beispielsweise eine Innenraumform für kelch-, parabel- oder kalottenartig geformte Enden 5 des Schaftes 6 der Endoprothese. Andere Außenformen des Schaftes 6 wie z.B. kegel-, kegelstumpf- oder kreissegmentartige Flächen oder aus Kombinationen dieser Flächen zusammengesetzte Formen gehören ebenso zur Erfindung. Die Probehülse 2 besteht aus einem halbzylinderförmig ausgebildeten Körper, bei dem die Innenraumform 4 entlang einer in Richtung der Zylinderachse ZA verlaufenden Ebene offen ist, so dass der Chirurg die Lage des in den Innenraum 3 eingeschobenen Endes 5 des Schaftes 6 einsehen und diejenige Probehülse leicht bestimmen kann, bei der die Außenform des Endes 5 des Schaftes 6 eine weitgehende Übereinstimmung mit der Innenraumform 4 zeigt.
An der distal geschlossenen Probehülse 2 befindet sich ein gerändelter Rand 14, der das Aufschieben und Drehen der Probehülse 2 auf das Ende 5 des Schaftes 6 erleichtert.

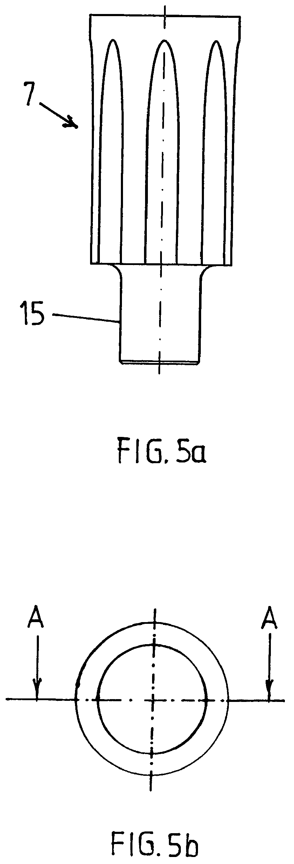

Beispiele für Implantatshülsen 7 zeigen die Schnittdarstellungen Fig. 6a bis 6c und 7a bis 7c unter Bezug auf die Fig. 5a und 5b, in deren Innenräume 8 jeweils die Enden 5 des Schaftes 6 der Endoprothese einzementiert sind. Die kraftschlüssige Einzementierung mit Knochenzement 24 ist entlang der Schnittkontur der Innenraumform der Implantatshülse durch einen schwarzen Rand angedeutet.
Die in Fig. 6a bis 6c gezeigte Implantatshülse 7 besitzt einen distal angeordneten Steckzapfen 15, dessen Außendurchmesser ADS an den Innendurchmesser ID der Durchführung 13 angepasst ist.
Dies ermöglicht es, die Halbschalen 12.1 und 12.2 ausschließlich gegen den Steckzapfen 15 zu spannen, so dass keine Spannkräfte auf das in im Innenraum 8 der Implantatshülse 7 einzementierte Ende 5 des Hüftschaftes übertragen werden.
Der Außendurchmesser ADI des übrigen Teils der zylindrischen Implantatshülse 7 entspricht im Wesentlichen dem Außendurchmesser ADD der Durchführung 13.
Die Fig. 7a bis 7c zeigen eine alternativ ausgebildete zylindrische Implantatshülse 7 mit gleichmäßigem Außendurchmesser ADI, in der die Einzementierung des Endes 5 des Schaftes 6 wie in den Fig. 6a bis 6c gekennzeichnet ist. In einem solchen Fall liegt die Implantatshülse 7 über ihre gesamte Länge L in der durch die Halbschalen 12.1 und 12.2 gebildeten Durchführung 13 ein, wodurch das einzementierte Ende 5 des Schaftes 6 der Prothese gleichmäßig von den Spannkräften beaufschlagt wird.
Natürlich ist aber auch möglich, dass nur das distale Ende 16 der zylindrischen Implantatshülse 7 in der Durchführung 13 positioniert wird, so dass auf den einzementierten Bereich des Endes 5 des Schaftes 6 lediglich geringfügige Einspannkräfte wirken.

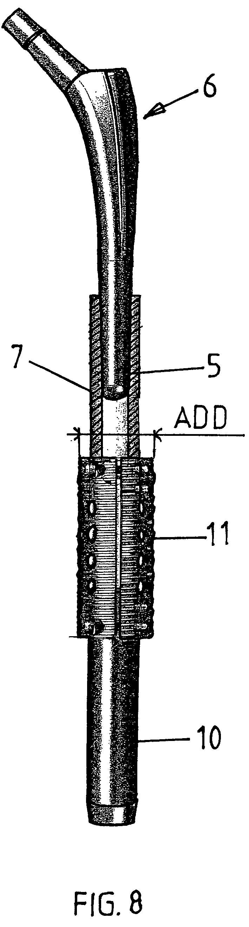

Die Figur 8 zeigt das erfindungsgemäße Adapter-System im zusammengebauten Zustand, in dem das einzementierte Ende 5 des Schaftes 6 außerhalb des Einspannbereichs der Halbschalen 12.1 und 12.2 liegt.

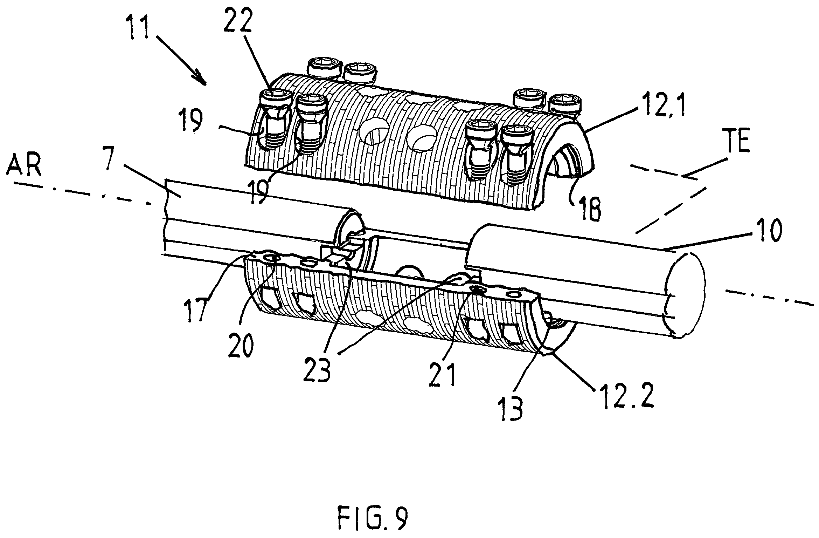

In der Fig. 9 ist das Verbindungsmittel 11 für die Implantatshülse 7 und den Nagel 10 dargestellt. Wie bereits in Abschnitt [0027] erwähnt, besteht das Verbindungsmittel 11 aus den zylindrischen Halbschalen 12.1 und 12.2, die im Wesentlichen gleichartig ausgebildet sind. Beide Halbschalen 12.1 und 12.2 definieren im zusammengesetzten Zustand die Durchführung 13, deren innere Wandung 17 eine senkrecht zur Achsrichtung AR der Halbschalen 12.1 und 12.2 gelegene Profilierung 18 besitzt. In die obere Halbschale 12.1 sind jeweils randseitig in Achsrichtung AR zwei nebeneinander angeordnete Ausnehmungen 19 so eingebracht, dass die Ausnehmungen 19 koaxial zur Achsrichtung AR und senkrecht zu den von den Halbschalen 12.1 und 12.2 virtuell aufgespannten Teilungsebene TE angeordnet sind. Jeder Ausnehmung 19 ist in der Wandung 17 der unteren Halbschale 12.2 eine entsprechend positionierte Bohrung 20 mit Innengewinde 21 zugeordnet, in das durch die Ausnehmungen 19 geführte Inbusschrauben 22eingeschraubt werden können. Die Halbschalen 12.1 und 12.2 bilden mit den Imbusschrauben 22 und den jeweiligen korrespondierenden Innengewinden 21 das Spannmittel, das sich beim Anziehen der Imbusschrauben um die Implantatshülse 7 und den um den Nagelhals 9 legt und in Abhängigkeit des Anzugsmomentes der Imbusschrauben 21 eine entsprechende kraftschlüssige Verbindung erzeugt.
Der Außendurchmesser ADS des Steckzapfens 15 bzw. der Außendurchmesser ADI der zylindrischen Implantatshülse 7 und der Außendurchmesser ADN des Nagelhalses 9 des Nagels 10 sind aufeinander entsprechend abgestimmt.
Die Imbusschrauben 21 sind nach ventral ausgerichtet und so einer späteren Revision problemlos von außen zugänglich, ohne große Bereiche des körpereigenen Gewebes traumatisieren zu müssen.
Die Halbschalen können unterschiedliche Längen L1, L2 besitzen oder mittels Modulverbinder verlängert werden, je nach dem wie es die Versorgung der periprothetischen Fraktur erfordert.
Die obere und untere Halbschale 12.1 bzw. 12.2 weist -wie Fig. 9 verdeutlicht- innerhalb der Durchführung 13 proximal- und distalseitig jeweils einen senkrecht von der Wandung 17 aufragenden Anschlag 23 für das Ende 5 des Hüftschaftes 6 der Endoprothese und für den Nagelhals 9 des Nagels 10 auf, wodurch eine gleichbleiende Stecktiefe für die Implantatshülse 7 und den Nagelhals 9 in der Durchführung 13 erreicht wird.

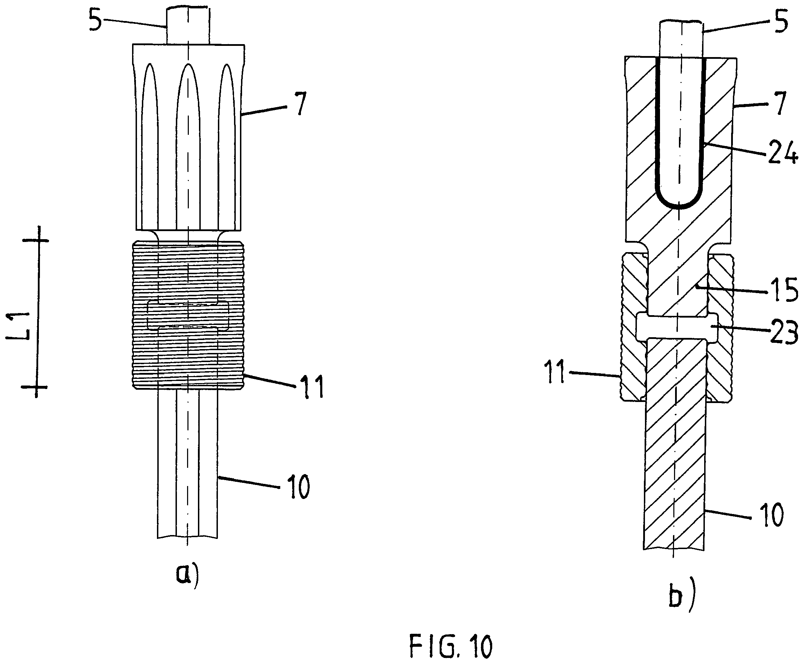

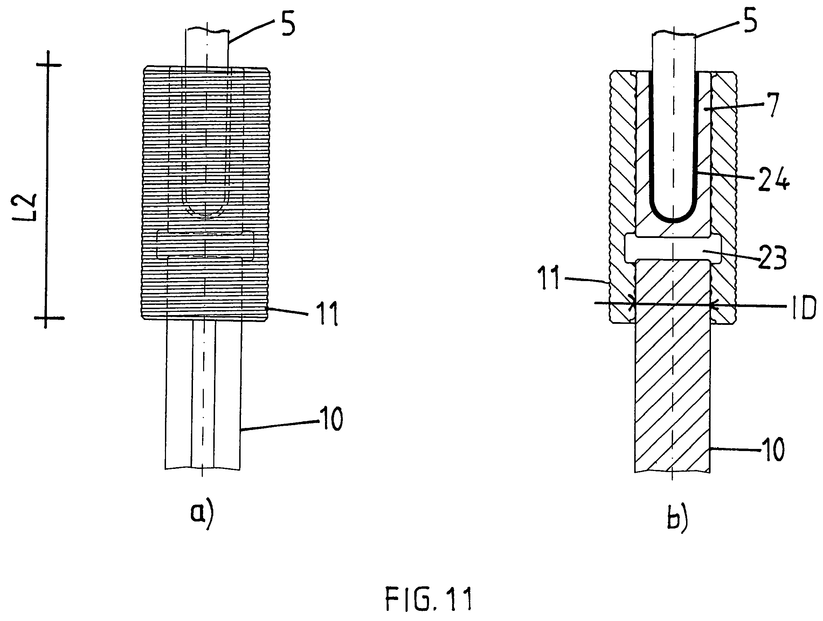

Die Fig. 10a, 10b, 11a und 11b zeigen zwei unterschiedlich lange Halbschalen in Draufsicht bzw. Schnittdarstellung, wobei die Halbschalen mit der kürzeren Länge L1 für eine Implantatshülse 7 mit Steckzapfen 15 und die Halbschalen mit der größeren Länge L2 für eine zylindrische Implantatshülse 7 mit gleichbleibendem Au0endurchmesser ADI vorgesehen ist.
In die Halbschalen 12.1 und 12.2 ist für die Implantatshülse 7 und den Nagelhals 9 des Nagels 10 ein gemeinsamer Anschlag 22 vorgesehen.

Alle Teile des erfindungsgemäßen Adapter-System bestehen mit Ausnahme der Imbusschrauben aus Titan, Niob oder deren Legierungen bzw. Implantatskunststoffe wie Peek.

Die Erfindung beschränkt sich in ihrer Ausführung nicht auf das vorgenannte Ausführungsbeispiel. Vielmehr sind Varianten denkbar, welche grundsätzlich andere Ausführungen beinhalten können. Die Erfindungen lässt sich problemlos für Frakturen in unterschiedlichen Bereichen wie z.B. im Bereich der Hüfte, des Knies, der Schulter oder anderen Endoprothesen mit Schaft einsetzen.

### Bezugszeichenliste

- Adapter-System: 1
- Prüfhülsen: 2
- Innenraum von 2: 3
- Innenraumgeometrie von 3: 4
- Ende von 6: 5
- Schaft der Endoprothese: 6
- Implantatshülsen: 7
- Innenraum von 7: 8
- Nagelhals von 10: 9
- Nagel: 10
- Verbindungsmittel: 11
- Obere Halbschale von 11: 12.1
- Untere Halbschale von 11: 12.2
- Durchführung: 13
- Gerändelter Rand von 2: 14
- Steckzapfen an 7: 15
- Distales Ende von 7: 16
- Innere Wandung von 12.1, 12.2: 17
- Profilierung: 18
- Ausnehmungen in 12.1: 19
- Bohrungen in 12.2: 20
- Innengewinde in 20: 21
- Imbusschrauben: 22
- Anschlag: 23
- Knochenzement: 24
- Achsrichtung von 11: AR
- Außendurchmesser von 7: ADI
- Außendurchmesser von 11: ADD
- Durchmesser des Nagelhalses: ADN
- Durchmesser von 15: ADS
- Innendurchmesser von 13: ID
- Länge von 7: L
- Länge von 12.1, 12.2: L1, L2
- Teilungsebene: TE
- Zylinderachse: ZA

## Patentansprüche

1. Periprothetisches Adapter-System für einen implantierten Schaft (6) einer Endoprothese, **dadurch gekennzeichnet, dass** das Adapter-System (1) umfasst:
a) einen Satz einer Vielzahl von zylindrischen, hohl ausgebildeten Probehülsen (2) mit jeweils unterschiedlichen, wenigstens hälftig in Richtung Zylinderachse (ZA) angeschnittenen Innenraumformen (4) zum Ermitteln der mit der Außenform des distalen Endes (5) des Schaftes (6) weitgehend übereinstimmenden Geometrie,
b) einen Satz von distal geschlossenen, aber proximal offenen mit dem Satz von Probehülsen (2) mindestens in der Innenraumform (4) übereinstimmenden Implantathülsen (7) zur Auswahl der geeigneten Implantathülse für die Aufnahme und eine kraftschlüssige Einbettung des distalen Endes (5) des Schaftes (6) in die Implantathülse mittels Knochenzement (24),
c) einen in den Knochen eingesetzten Nagel (10) mit einem zylindrischen Nagelhals (9) und
d) ein Verbindungsmittel (11) für das in die Implantathülse (7) einzementierte Ende (5) des Schaftes (6) und den Nagel (10) in Form eines an sich bekannten Moduls aus mindestens zwei in Achsrichtung des Moduls geteilte, lösbar miteinander verbundene Halbschalen (12.1,12.2) die miteinander eine sich axial erstreckende Durchführung (13) zum Einführen des distalen Endes (16) der Implantathülse (7) und des proximalen Nagelhalses (9) ausbilden, wobei die Halbschalen (12.1, 12.2) durch senkrecht zur Achsrichtung (AR) positionierte Spannmittel den Nagelhals (9) und die Implantathülse (7) in der Durchführung (13) spannbackenartig fixieren.

2. Periprothetisches Adapter-System nach Anspruch 1, **dadurch gekennzeichnet, dass** der Außendurchmesser (AD) der Implantatshülse (7) und der Außendurchmesser (ADN) des zylindrischen Nagelhalses (9) an den Innendurchmesser (ID) der Durchführung (13) der Halbschalen (12.1, 12.29) angepasst ist.

3. Periprothetisches Adapter-System nach Anspruch 1, **dadurch gekennzeichnet, dass** die Implantatshülsen (7) unterschiedliche Außendurchmesser (ADI) aufweisen.

4. Periprothetisches Adapter-System nach Anspruch 1, **dadurch gekennzeichnet, dass** die Implantatshülsen (7) unterschiedlich axiale Längen, Außendurchmesser und Innenraumformen aufweisen, denen jeweils die Prüfhülsen zum Ermitteln eines formschlußnahen Sitzes des Endes (5) des Schaftes (6) in der Implantatshülse (7) zugeordnet sind

5. Periprothetisches Adapter-System nach Anspruch 1, **dadurch gekennzeichnet, dass** die Implantatshülsen (7) distal einen Steckzapfen (15) aufweisen.

6. Periprothetisches Adapter-System nach Anspruch 1, **dadurch gekennzeichnet, dass** die Innenräume der Probe- und Implantatshülsen (2, 7) eines Satzes mit Innenraumformen versehen sind , die in Form und Geometrie an das Schaftende (6) handelsüblicher Endoprothesen angepasst sind.

7. Periprothetisches Adapter-system nach Anspruch 6, **dadurch gekennzeichnet, dass** die Innenraumform vorzugsweise aus zylindrischen, kegel-, kegelstumpf-, parabel-, kreissegment-, halbkugelförmigen Flächen oder aus entsprechend zusammengesetzten Flächen gebildet ist.

8. Periprothetisches Adapter-System nach Anspruch 1, **dadurch gekennzeichnet, dass** die Innenraumform (4) eine aufgeraute Oberfläche zum Verbessern der Haftkraft des Knochenzementes aufweist.

9. Periprothetisches Adapter-System nach Anspruch 1, **dadurch gekennzeichnet, dass** die Halbschalen (12.1, 12.2) längenvariabel sind und eine an periprothetische Frakturen individuell anpassbare Längenvariabilität erreichen.

10. Periprothetisches Adapter-System nach Anspruch 1, **dadurch gekennzeichnet, dass** zum Verbinden von Modulen gleicher oder unterschiedlicher Länge ein Modulverbinder vorhanden ist, dessen Außendurchmesser auf den Innendurchmesser (ID) der Durchführung (13) abgestimmt ist.

11. Periprothetisches Adapter-System nach Anspruch 1, **dadurch gekennzeichnet, dass** der Durchmesser der Module unterschiedlicher Länge gleich ist.

12. Periprothetisches Adapter-System nach Anspruch 1, **dadurch gekennzeichnet, dass** zum Anpassen des Durchmessers des Nagelhalses auf den Innendurchmesser (ID) der Durchführung (13) eine Reduzierbuchse vorgesehen ist.

13. Periprothetisches Adapter-System nach Anspruch 1, **dadurch gekennzeichnet, dass** alle Teile des Adapter-Systems aus einem körperverträglichen und beständigen, vorzugsweise metallischen Werkstoff, beispielsweise Titan, Tantal, Niob oder deren Legierungen bzw. moderne Implantatskunststoffe wie Peek bestehen.

## Claims

1. A periprosthetic adapter system for an implanted stem (6) of an endoprosthesis, **characterised in that** the adapter system (1) comprises:
a) a set of a plurality of cylindrical, hollow test sheaths (2) in each case with different interior shapes (4) cut at least halfway in the direction of the cylinder axis (ZA) for determining the geometry largely corresponding to the external shape of the distal end (5) of the stem (6),
b) a set of distally closed but proximally open implant sheaths (7) corresponding to the set of test sheaths (2) at least in interior shape (4) for selection of the suitable implant sheath for accommodating and embedding the distal end (5) of the stem (6) in noninterlocking manner in the implant sheath using bone cement (24),
c) a nail (10) inserted into the bone and having a cylindrical nail neck (9) and
d) a connecting means (11) for the end (5) of the stem (6) cemented into the implant sheath (7) and the nail (10) in the form of a module known per se composed of at least two half-shells (12.1, 12.2) which are divided in the axial direction of the module and connected detachably together and which together form an axially extending passage (13) for insertion of the distal end (16) of the implant sheath (7) and of the proximal nail neck (9), the half-shells (12.1, 12.2) fixing the nail neck (9) and the implant sheath (7) in the passage (13) in the manner of clamping jaws by clamping means positioned perpendicular to the axial direction (AR).

2. A periprosthetic adapter system according to claim 1, **characterised in that** the external diameter (AD) of the implant sheath (7) and the external diameter (ADN) of the cylindrical nail neck (9) is adapted to the internal diameter (ID) of the passage (13) of the half-shells (12.1, 12.2).

3. A periprosthetic adapter system according to claim 1, **characterised in that** the implant sheaths (7) have different external diameters (ADI).

4. A periprosthetic adapter system according to claim 1, **characterised in that** the implant sheaths (7) have different axial lengths, external diameters and interior shapes, to which are in each case assigned the test sheaths for establishing a virtually interlocking fit of the end (5) of the stem (6) in the implant sheath (7)

5. A periprosthetic adapter system according to claim 1, **characterised in that** the implant sheaths (7) comprise an insertion pin (15) at the distal end.

6. A periprosthetic adapter system according to claim 1, **characterised in that** the interiors of the test and implant sheaths (2, 7) of a set are provided with interior shapes which are adapted in shape and geometry to the stem end (6) of conventional commercial endoprostheses.

7. A periprosthetic adapter system according to claim 6, **characterised in that** the interior shape is preferably formed from cylindrical, conical, truncated cone-shaped, parabolic, circular segment-shaped or hemispherical surfaces or of appropriately composed surfaces.

8. A periprosthetic adapter system according to claim 1, **characterised in that** the interior shape (4) has a roughened surface for improving the adhesive force of the bone cement.

9. A periprosthetic adapter system according to claim 1, **characterised in that** the half-shells (12.1, 12.2) are variable in length and achieve a variability of length individually adaptable to periprosthetic fractures.

10. A periprosthetic adapter system according to claim 1, **characterised in that,** to connect modules of identical or different length, a module connector is provided, the external diameter of which is matched to the internal diameter (ID) of the passage (13).

11. A periprosthetic adapter system according to claim 1, **characterised in that** the modules of different length are of identical diameter.

12. A periprosthetic adapter system according to claim 1, **characterised in that** a reducing bushing is provided to adapt the diameter of the nail neck to the internal diameter (ID) of the passage (13).

13. A periprosthetic adapter system according to claim 1, **characterised in that** all the parts of the adapter system consist of a biocompatible, durable, preferably metallic material, for example titanium, tantalum, niobium or the alloys thereof or modern implant plastics such as PEEK.

## Revendications

1. Système d'adaptateur périprothétique pour une tige implantée (6) d'une endoprothèse, **caractérisé en ce que** le système d'adaptateur (1) comprend :
a) un ensemble d'une pluralité de manchons d'essai (2) cylindriques creux présentant des formes d'espace intérieur (4) différentes, découpées au moins à moitié en direction de l'axe de cylindre (ZA), pour déterminer la géométrie concordant en grande partie avec la forme extérieure de l'extrémité distale (5) de la tige (6),
b) un ensemble de manchons d'implant (7) fermés du côté distal mais ouverts du côté proximal, concordant avec l'ensemble de manchons d'essai (2) au moins en ce qui concerne la forme d'espace intérieur (4), pour sélectionner le manchon d'implant approprié pour la réception et un encastrement à force de l'extrémité distale (5) de la tige (6) dans le manchon d'implant au moyen d'un ciment osseux (24),
c) un clou (10) introduit dans l'os et pourvu d'un col (9) cylindrique, et
d) un moyen de liaison (11) pour l'extrémité (5) de la tige (6) cimentée dans le manchon d'implant (7) et le clou (10), sous forme d'un module en soi connu constitué d'au moins deux demi-coquilles (12.1, 12.2) divisées dans le sens axial du module et reliées entre elles de manière détachable, qui forment ensemble un passage (13) s'étendant axialement pour introduire l'extrémité distale (16) du manchon d'implant (7) et le col proximal (9) du clou, les demi-coquilles (12.1, 12.2) fixant à la manière de mâchoires de serrage le col (9) du clou et le manchon d'implant (7) dans le passage (13), grâce à des moyens de serrage positionnés perpendiculairement au sens axial (AR).

2. Système d'adaptateur périprothétique selon la revendication 1, **caractérisé en ce que** le diamètre extérieur (AD) du manchon d'implant (7) et le diamètre extérieur (ADN) du col cylindrique (9) du clou sont adaptés au diamètre intérieur (ID) du passage (13) des demi-coquilles (12.1, 12.2).

3. Système d'adaptateur périprothétique selon la revendication 1, **caractérisé en ce que** les manchons d'implant (7) ont des diamètres extérieurs (ADI) différents.

4. Système d'adaptateur périprothétique selon la revendication 1, **caractérisé en ce que** les manchons d'implant (7) ont des longueurs axiales, diamètres extérieurs et formes intérieurs différents auxquels sont associés les manchons d'essai respectifs pour déterminer une mise en place proche d'un ajustement par complémentarité de forme de l'extrémité (5) de la tige (6) dans le manchon d'implant (7).

5. Système d'adaptateur périprothétique selon la revendication 1, **caractérisé en ce que** les manchons d'implant (7) ont du côté distal un tenon d'emboîtement (15).

6. Système d'adaptateur périprothétique selon la revendication 1, **caractérisé en ce que** les espaces intérieurs des manchons d'essai et d'implant (2, 7) d'un ensemble présentent des formes d'espace intérieur dont la forme et la géométrie sont adaptées à l'extrémité de la tige (6) d'endoprothèses disponibles dans le commerce.

7. Système d'adaptateur périprothétique selon la revendication 6, **caractérisé en ce que** la forme d'espace intérieur est formée de préférence par des surfaces cylindriques, coniques, tronconiques, paraboliques, en segment de cercle, hémisphériques ou par des surfaces assemblées de façon correspondante.

8. Système d'adaptateur périprothétique selon la revendication 1, **caractérisé en ce que** la forme d'espace intérieur (4) présente une surface rugueuse pour améliorer la force adhésive du ciment osseux.

9. Système d'adaptateur périprothétique selon la revendication 1, **caractérisé en ce que** les demi-coquilles (12.1, 12.2) sont variables en longueur et atteignent une variabilité en longueur individuellement adaptable aux fractures périprothétiques.

10. Système d'adaptateur périprothétique selon la revendication 1, **caractérisé en ce que,** pour relier deux modules de même longueur ou de longueur différente, il est prévu une jonction de modules dont le diamètre extérieur est adapté au diamètre intérieur (ID) du passage (13).

11. Système d'adaptateur périprothétique selon la revendication 1, **caractérisé en ce que** le diamètre des modules de longueur différente est le même.

12. Système d'adaptateur périprothétique selon la revendication 1, **caractérisé en ce que,** pour adapter le diamètre du col du clou au diamètre intérieur (ID) du passage (13), il est prévu une douille de réduction.

13. Système d'adaptateur périprothétique selon la revendication 1, **caractérisé en ce que** toutes les parties du système d'adaptateur sont faites d'un matériau toléré par le corps et résistant, de préférence métallique, tel que le titane, le tantale, le niobium ou leurs alliages, ou bien de matières plastiques pour implants comme le PEEK.
